Europäisches Patentamt

European Patent Office

Office européen des brevets

(19)

(11) Publication number: **0 097 633**
**B1**

(12) **EUROPEAN PATENT SPECIFICATION**

(45) Date of publication of patent specification: **28.05.86**

(21) Application number: **83850174.0**

(22) Date of filing: **21.06.83**

(51) Int. Cl.⁴: **G 01 N 33/569,**
**A 61 K 39/395, C 12 Q 1/70**

(54) **Determination of the sensitivity of virus to antiviral agents.**

(30) Priority: **23.06.82 SE 8203909**

(43) Date of publication of application:
**04.01.84 Bulletin 84/01**

(45) Publication of the grant of the patent:
**28.05.86 Bulletin 86/22**

(84) Designated Contracting States:
**BE CH DE FR GB LI NL**

(56) References cited:
**US-A-4 297 104**

(73) Proprietor: **Wahren, Britta**
**Frithjofsvägen 10**
**S-18264 Djursholm (SE)**
(73) Proprietor: **Harmenberg, Johan**
**Öregrundsgatan 14**
**S-115 28 Stockholm (SE)**
(73) Proprietor: **Sundqvist, Vivi-Anne**
**Sotingeplan 8**
**S-16361 Spänga (SE)**

(72) Inventor: **Wahren, Britta**
**Frithjofsvägen 10**
**S-18264 Djursholm (SE)**
Inventor: **Harmenberg, Johan**
**Öregrundsgatan 14**
**S-115 28 Stockholm (SE)**
Inventor: **Sundqvist, Vivi-Anne**
**Sotingeplan 8**
**S-16361 Spänga (SE)**

(74) Representative: **Onn, Thorsten et al**
**AB STOCKHOLMS PATENTBYRA Box 3129**
**S-103 62 Stockholm (SE)**

Courier Press, Leamington Spa, England.

# 0 097 633

**Description**

This invention relates to determination of the sensitivity of virus to various antiviral agents.

Herpes virus infections are among the most usual virus diseases. After the primary infection, which is often asymptomatic, a latent infection with remaining virus is established. Herpes virus is then probably often reactivated, sometimes subclinically, but sometimes with cell destruction and damage of organs as a consequence. A series of external factors such as irradiation, medicaments or another virus infection — often actually another herpes virus — can give such a reactivation. Paradoxically enough, the frequency and the difficulty of reactivated herpes infections will increase the more sophisticated other methods of treatment are provided for medical care. An intensive — often life-saving — chemotherapy treatment of malign tumor diseases, as well as radiation treatment unintentionally also affecting cells with a latent virus (nerve ganglions, blood cells) can provoke an outbreak of herpes infections.

Herpes simplex types 1 and 2, varicella-zoster, cytomegalovirus and Epstein-Barr-virus are counted among the herpes virus normally infecting man. Patients with herpes virus infections are increasing in number in medical care. During intensive treatment with chemotherapy, radiation treatment and transplantation, virus are easily reactivated in these less resistant patients. A herpes infection may result in serious conditions and even be lethal to these patients which are often successfully treated for some other disease.

However, certain virus diseases, especially herpes virus infections, have recently been treated with success with antiviral agents. A prerequisite of an adequate treatment both in respect of an antiviral agent for treatment and the length of treatment time is however, that the type of virus is determined and that the sensitivity to different possible antiviral agents is determined.

The sensitivity of a virus to antiviral agents can be determined by means of conventional techniques using methods comprising replication of virus in cells, cultivation, and repeated replication of virus in cells with addition of different amounts and types of antiviral agents followed by plaque-counting. This method taking at least 7—14 days depending on type of virus is time-consuming, complicated and expensive. (Crumpacher, C. C., Schnipper, L. E., Zaia, J. A., Levin, M. J.: "Growth inhibition by acycloguanosine of herpes viruses isolated from human infections." Antimicrob. Agents Chemother. 15, 642, 1979. Svennerholm, B., Vahlne, A., Lycke, E. "Inhibition of Herpes simplex virus infection in tissue culture by trisodium phosphonoformate." Proc. Soc. Exp. Biol. Med., 61, 115—118, 1979.)

It is consequently the object of the invention to develop a method of determining the sensitivity of virus to different antiviral agents, which method is simpler, more economical and, above all, faster than conventional technique.

This object is achieved according to the invention by means of a method for determining the sensitivity of a virus to an anti-viral agent through

a) inoculation of a viral isolate thereof or a material containing such a viable virus mixed with tissue culture medium in batches of cells which permit virus replication;

b) addition of varying concentrations of the antiviral agent to the cells;

c) incubation of the inoculated cells, to which an antiviral agent has been added, and of at least one batch of inoculated cells to which the antiviral agent has not been added, after which the virus is allowed to replicate; and

d) measurement of the viral replication in the cells, characterized in that, before the measurement d) the cells are disrupted by ultrasonication, hypertonic solutions, mechanical or chemical disintegration and/ or freeze-thawing in the culture medium or after removal of the supernatant followed by addition of distilled water, thereby allowing measurement of the virus antigen content of the cells before or after occurrence of a cytopathic effect in the measurement step d); and in that the measurement d) is carried out by immunological determination of the amount of viral antigen formed through incubation, said determination including a virus antigen-antibody reaction; whereafter

e) the viral sensitivity to the antiviral agent is determined by a comparison of the viral replication obtained by the incubation of the virus cells with addition of the antiviral agent with the viral replication obtained by the incubation of the cells without addition of the antiviral agent.

The method of the invention can be denominated by the rational name virus sensitivity assay (VSA).

The invention is described in more detail in the following with reference to herpes virus. However, the invention is not restricted to herpes virus but is applicable to any virus which can replicate in vitro in a cell culture. The invention is especially intended to be used for such virus for which antiviral agents have already been developed or may be developed in the near future.

When carrying out the method of the invention cells are first inoculated with a mixture of a virus-isolate or a virus containing material usually deriving from material from a patient containing alive virus, e.g. material from a skin blister, urine, throat washing fluid or lachrymal secretion, a small amount of tissue culture medium and usually also antibiotics, such as penicillin and streptomycin, e.g. 100 IE/ml and 100 µg/ ml, respectively, to suppress bacterial growth. After dilution, preferably 1:30 for herpes virus and 1:5 for cytomegalovirus and varicella-zoster virus, this virus mixture can be inoculated directly in cells, preferably using sterile plastic plates with cell-containing wells (holes). Alternatively the virus mixture can first be cultured in cells allowing virus replication, e.g. human fibroblasts, after which the virus obtained after one cell passage is investigated. This alternative can be suitable if only a small amount of virus containing

2

material is at hand. A suitable tissue culture medium is Eagle's tissue culture medium containing a minor amount, e.g. 2%, of calf serum. Another useful tissue culture medium is RPMI 1640.

The cells on which the virus mixture is inoculated are those that permit virus replication and which can be cultured in a tissue culture medium. Human lung fibroblasts (HL-cells) allow replication of herpes simplex virus (HSV), cytomegalovirus (CMV) and varicella-zoster virus (VZV). These viruses are the human herpes viruses treated at present with antiviral agents. Of course other cell types permitting replication of the virus to be investigated can also be used, e.g. Vero cells or GMK cells.

After the cells have been inoculated with the virus-containing mixture different concentrations of the antiviral substance are added to different batches of cells, e.g. to cells in different wells on the above-mentioned sterile plastic plates. The concentration is preferably a dilution series with a dilution factor of 1:n, where n is a number, e.g. with a value of 2—10. Of course several different antiviral agents can then be tested starting from the same virus containing material of a patient if a sufficient number of batches of cells, e.g. cell-containing wells in the plastic plates, are used. Virus are allowed to replicate without addition of any antiviral substance to at least one batch of cells inoculated with the virus containing material. Moreover, it is suitable that a detergent inactivated virus-containing material is added to one or more batches of cells for virus typing and as a background control that virus replication has taken place in the other cell batches. Detergent inactivation of virus means that virus cannot be replicated, but virus antigen can still be established. A suitable detergent is Brij—58® (ICI America, Inc, Wilmington, DE, USA) or Triton X®.

The antiviral agents that can be evaluated according to the invention are by no means restricted but all those known at present and those as may be developed in future can be utilized in the method of the invention. Examples of antiviral agents known at present are acyclovir (ACV), phosphonoformic acid (PFA), iododeoxyuridine (IDU), adenosine-arabinoside (Ara-A) and interferon (IF), which can be used for treatment of herpes simplex type 1 and 2 and varicella-zoster. For cytomegalovirus PFA and different types of interferons are used. Also immunoglobulins are antiviral agents.

In the following immunological determination of the virus replication carried out for evaluation of the effect of the added antiviral agents, the virus replication is measured indirectly by determining the amount of formed virus antigens utilizing antibody-antigen interaction. The amount of late formed antigens, which are not formed until virus nucleic acid has replicated, is preferably measured.

The time of the immunological determination is not only dependent on the virus type but also on whether a high or low virus concentration is obtained after the replication in the cells. If a high or low virus concentration can be expected it can e.g. be read visually by a microscope on the basis of the cytopathic effect in cells inoculated without addition of an antiviral agent. A cytopathic effect in the cells is however, not required for antigen measurement. The immunological determination is preferably carried out when an optimal virus antigen content has been obtained, as it has been found that the differentiation between different antiviral agents appears most distinctly in ELISA® at A 405 mm = 0.8—1.5 (see below). At a high content of virus the immunological determination should with HSV be carried out as early as after 24 hours since the cells are rapidly destroyed and measureable antigen decreases probably due to degradation. At an expected lower virus content the virus replication should be interrupted after about 2—3 days in order to achieve the best measuring result in the immunological determination. For CMV and VZV the corresponding time is 3—5 days.

Except for the cell disrupture carried out before the immunological determination characterizing the invention and which will be explained more in detail below, the choice of immunological determination method is essential in order to obtain good results by the method of the invention. A very sensitive method must be chosen. Several such methods are described in literature in this field and the invention is not restricted to any special such determination method. However, among the immunological methods utilized at present, especially enzyme immunological methods based on ELISA® (enzyme linked immunosorbent assay) are utilized, although radioimmunological methods based on RIA (radioimmunoassay) can give acceptable results for certain purposes.

RIA as well as ELISA® are methods well-known to one skilled in the art and described in the available literature. RIA comprises utilization of radioactive labelling of e.g. an antibody, usually with radioactive iodine and measurement of the amount of free and antigen-bonded, respectively, labelled antibody. For separation of a free antibody from antigen-bonded antibody the principles of immunosorbence are often utilized, i.e. utilization of an antigen or antibody made insoluble with maintained reactivity, usually by covalent linkage to a solid matrix, such as particles of Sepharose® (Pharmacia Fine Chemicals, Uppsala, Sweden), or by physical adsorption to glass or plastic surfaces. For ELISA the same principles as for RIA are utilized. For detection, an enzyme labelled conjugate is utilized and once the antigen-antibody interaction is completed, the addition of a substrate allows the assessment of enzyme activity by colorimetrically or fluorimetrically measuring the formed reaction product. One advantage of ELISA in addition to its great sensitivity permitting determination of very low concentrations of virus antigen, is that enzyme-labelled reagents have a long durability and that relatively cheap measuring equipment can be used.

A method based on ELISA and which is especially adapted for the determination of virus antibodies and virus antigens and which is preferably used for the immunological determination according to the invention is described in Journal of Virological Methods, 2 (1981), pages 301—312. The method is especially described with reference to CMV but can be utilized as well for other virus, especially other virus of the herpes group. Inhibition technique in which the antigen-containing sample is incubated with antibodies of one single type as well as sandwich technique in which the incubation is carried out with two different types of antibodies can then be utilized. The sandwich technique is preferably used since lower antigen quantities can be detected.

In order that the sensitivity of a virus to various antiviral agents might be determined with a great accuracy it is not enough that materials from a patient which contain the virus are incubated in cells, as described above, and that in the supernatant the amount of virus antigen formed after virus replication is measured by means of a very sensitive immunological method, such as the enzyme immunological method described above.

According to the invention it has surprisingly been found that the sensitivity of the method can be increased essentially if the cells are treated before the immunological determination so that cell disruption is achieved, which means that the virus antigen content of the cells is measured in the following immunological determination instead of virus antigen in the supernatant.

This cell disruption can be achieved by ultrasonication, hypertonic solutions or another mechanical or chemical disintegration. Preferably it is carried out by addition of distilled water and freeze thawing, e.g. in the following way:

The supernatant is eliminated from the cell batches inoculated and incubated with a virus containing material with or without addition of an antiviral agent, and distilled water is added to the cells. This must be carried out as soon as possible after the eclipse time of virus so that no cytopathic effect goes too far or cell disintegration occurs. The cells are thereafter frozen at a temperature which is low enough and for a time long enough for ice crystals to form. A temperature range of $-5°C$ to $-70°C$ can be used, $-20°C$ being a suitable temperature. The cells can be preserved for a long time, e.g. several months, in a frozen state before the determination of virus antigen. When the amount of virus antigen is to be determined the cells are thawed at room temperature, i.e. about 15—30°C, and a solution of NaCl, e.g. 4.5% NaCl, is added to achieve an isotonic solution. In the supernatant containing the virus antigen the amount of virus antigen can be measured immunologically, e.g. with ELISA sandwich technique. However, the best result is achived by measurement on cell scrap, i.e. cells which have been scraped off and by homogenization of the cell scrap in a detergent, e.g. 0.5% of Triton X or 1% Brij®—58, the yield can be increased a little further.

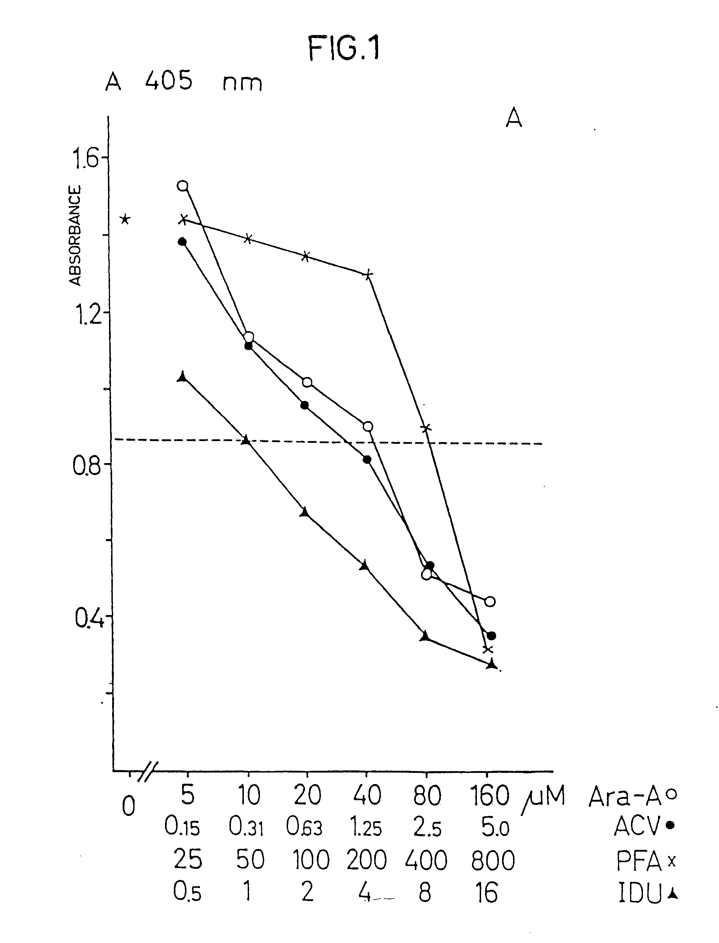

In the following examples the invention is explained more in detail. It is referred to the enclosed drawings, in which Figs 1—4 show absorbance values plotted against the concentration of an antiviral agent, which values are measured with ELISA-sandwich technique on virus infected HL-cells incubated in the presence of an antiviral agent.

### Example 1

A material from a patient containing living HSV is mixed with a small amount of Eagle's tissue culture medium containing 2% of calf serum, 100 IE penicillin/ml and 100 µg of streptomycin/ml, after which the virus mixture is diluted 1:30 in this medium.

Sterile plastic plates comprising 24 wells, each having a culture surface of 2.5 cm$^2$ and containing about $5 \times 10^5$ HL-cells/well were inoculated with 0.1 ml of virus dilutions as above. Such of the above virus dilutions only containing a small amount of virus are first cultured for 3 days in HL-cells, on which virus replicates, after which the virus isolate from one cell passage is taken out, is diluted 1:30 and 0.1 ml of the dilutions is added to the plates as indicated above. The cells are then incubated at a temperature of 32—37°C, with addition of different antiviral agents, and without any addition of antiviral agent to obtain reference values. Each antiviral agent is added in a dilution series comprising six different concentrations $(C_1—C_6)$, some of which correspond to desired serum concentrations for the treatment of patients. In table 1 is indicated the antiviral agents and concentrations thereof used.

As a background control of virus replication taking place and for a direct typing of virus 2 cell containing wells/plate are inoculated with a mixture of 0.1 ml virus dilution and 0.05 ml of Brij—58 of 1%.

TABLE 1
Concentrations of antiviral agents

|  | Serum concentrations used clinically µM or IU/ml | Concentrations used for HSV-inhibition in vitro µM or IU/ml | | | | | |
|---|---|---|---|---|---|---|---|
|  |  | $C_1$ | $C_2$ | $C_3$ | $C_4$ | $C_5$ | $C_6$ |
| Ara-A | 10—15 | 5 | 10 | 20 | 40 | 80 | 160 |
| ACV | 0.1—1.0 | 0.15 | 0.31 | 0.63 | 1.25 | 2.5 | 5 |
| PFA | 100 | 25 | 50 | 100 | 200 | 400 | 800 |
| IDU | 4—400[1] | 0.5 | 1 | 2 | 4 | 8 | 16 |

[1] The concentrations are measured in lachrymal fluid

4

After a culture of 1 to 3 days the effect of the antiviral agents is then evaluated by measurement with ELISA sandwich technique as described below. However, the cells are treated in the following way before this measurement.

The supernatant is sucked off and in order to achieve cell disrupture 800 µl of distilled water are added to the cells on the plates. The plates are frozen at −20°C and after some time sufficient for formation of ice crystals (about 15 min) the plates are thawed to room temperature for measurement of virus antigen. If desired, the plates can be preserved in a frozen state for a long time before they are thawed in order to carry out the measurement of virus antigen. When the plates have thawed 200 µl of NaCl of 4.5% are added to the cells in order to obtain an isotonic solution.

The amount of virus antigen formed is thereafter determined as follows. Guinea pig anti-HSV-serum is diluted in 0.1-M sodium carbonate buffer. 0.1 ml of the serum dilution is filled per well in microplates (M 29 AR, Dynatech, Zug, Switzerland) which have been washed three times with NaCl-solution of 0.9% containing 0.05% of Tween® 20, after which the plates are incubated for 16 hours at room temperature. The plates can be preserved sealed with their contents at +4°C up to 2—3 months. The optimal antibody concentration has been determined by antibody titration with the respective antigen and control antigen.

0.1 ml of the cell material obtained above or a known virus antigen is filled into plates treated with antibody and is incubated for 4 hours at +4°C. After washing with the washing solution indicated above 0.1 ml of rabbit anti-HSV is added and the plates are incubated for 2 hours at +37°C. The plates are washed and 0.1 ml of conjugate is added, after which they are incubated for 2 hours at +37°C. The conjugate consists of pig anti-rabbit IgG conjugated with alkaline phosphatase (Orion Diagnostica, Helsinki, Finland) in a dilution of 1:100. Non-bonded conjugate is washed off before 100 µl of the substrate p-nitrophenyl phosphate (1 mg/ml, Sigma, St Louis, MO, USA) are added. For dilution of the substrate 1 M diethanol buffer, pH 9.8 containing $0.5 \times 10^{-3}$ M $MgCl_2$ is used and for dilution of antisera and conjugate phosphate buffer (PBS), pH 7.3—7.4 with 0.5% of Tween® 20, 0.5% of BSA (bovine serum albumin) and 0.02% of $NaN_3$ is used. The reaction is stopped after 20—60 min with 0.025 ml of 5 M NaOH. The absorbance (A) is thereafter read at 405 nm with Titertek Multiskan® (Flow Laboratories, Irvine, Scotland). Absorbance values then obtained at the concentrations $C_1$—$C_6$ of the antiviral agents from table 1 and at the concentration $C_0 = O$ are indicated in the following table 2.

### TABLE 2

| Antiviral agent | Concentration of antiviral agent | | | | | | |
|---|---|---|---|---|---|---|---|
| | $C_0$ | $C_1$ | $C_2$ | $C_3$ | $C_4$ | $C_5$ | $C_6$ |
| Ara—A | 1.425 | 1.505 | 1.132 | 1.017 | 0.892 | 0.520 | 0.473 |
| ACV | " | 1.382 | 1.135 | 0.958 | 0.810 | 0.547 | 0.361 |
| PFA | " | 1.437 | 1.391 | 1.346 | 1.321 | 0.893 | 0.310 |
| IDU | " | 1.011 | 0.847 | 0.671 | 0.536 | 0.344 | 0.298 |

In fig. 1 these absorbance values have been plotted against the concentrations of $C_0$—$C_6$ for each antiviral agent. (The concentrations $C_1$—$C_6$ are then different for the various antiviral agents in agreement with table 1.). It is quite apparent from Fig. 1 that the investigated HSV shows a high sensitivity to ACV and IDU, but a lower one to PFA.

### Example 2

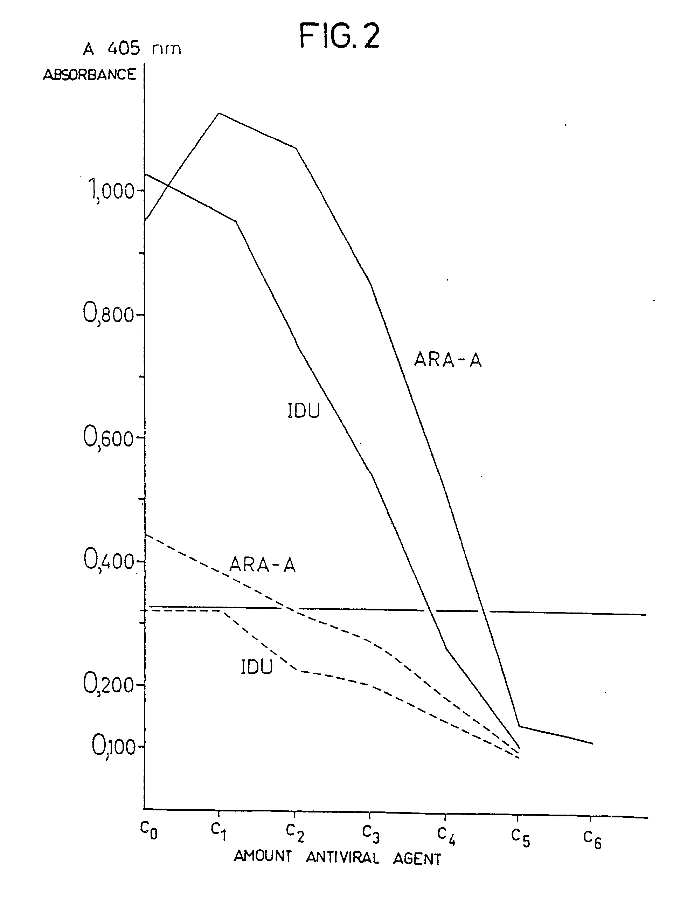

HL-cells infected with HSV—1 were incubated in the same way as in example 1 in the presence of Ara-A and IDU in the concentrations indicated in table 1 for 3 days. Reference culture without addition of antiviral agent was also carried out. The virus antigen in the supernatant (- - -) and in cells disrupted with distilled water (——) was thereafter measured in the same way as in example 1, and the results are found in table 3 and Fig. 2.

### TABLE 3
#### Absorbance values at 405 nm

| Antiviral agent | Concentration of antiviral agent | | | | | | |
|---|---|---|---|---|---|---|---|
| | $C_0$ | $C_1$ | $C_2$ | $C_3$ | $C_4$ | $C_5$ | $C_6$ |
| Ara-A cells | 0.948 ± 0.135 | 1.130 ± 0.052 | 1.073 ± 0.054 | 0.863 ± 0.030 | 0.513 ± 0.025 | 0.142 ± 0.009 | 0.119 |
| Ara-A supernatant | 0.448 ± 0.003 | 0.384 ± 0.025 | 0.323 ± 0.010 | 0.276 ± 0.006 | 0.184 ± 0.009 | 0.099 ± 0.006 | 0.099 |
| IDU cells | 1.025 ± 0.121 | 1.130 ± 0.052 | 1.075 ± 0.054 | 0.863 ± 0.030 | 0.513 ± 0.025 | 0.142 ± 0.009 | 0.119 |
| IBU supernatant | 0.320 ± 0.054 | 0.320 ± 0.037 | 0.259 ± 0.005 | 0.201 ± 0.025 | 0.142 ± 0.019 | 0.091 ± 0.017 | 0.143 |

It is apparent from Fig. 2 that the cells have a very high HSV-antigen content as compared with the supernatant and that 50% of inhibition of antigen formation in the cells are achieved both with IDU and ARA-A in clinically relevant concentrations.

It is apparent from table 3 that the standard deviation in each point is low, 5—10%.

#### Example 3

A virus isolate from an eye IDU treated with without result was tested in the same way as in example 1, the antiviral agents indicated in table 1 being used in the concentrations stated therein.

The following adsorbance values were measured (table 4).

### TABLE 4

| Antiviral agent | Concentration of antiviral agent | | | | | | |
|---|---|---|---|---|---|---|---|
| | $C_0$ | $C_1$ | $C_2$ | $C_3$ | $C_4$ | $C_5$ | $C_6$ |
| Ara-A | 1.605 | 1.572 | 1.482 | 0.750 | 0.155 | 0.071 | 0.039 |
| ACV | | 1.608 | 1.615 | 1.594 | 1.179 | 1.475 | 1.415 |
| PFA | | 1.487 | 1.502 | 0.656 | 0.216 | 0.092 | 0.083 |
| IDU | | 1.207 | 1.165 | 0.951 | 0.739 | 0.706 | 0.502 |

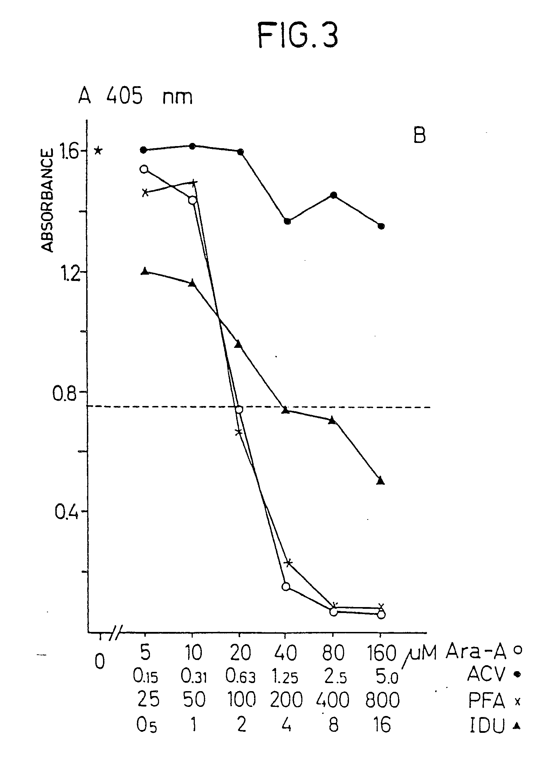

These values are given in Fig. 3, from which is apparent that the virus isolate has resistance to IDU and ACV and partial resistance to IDU.

#### Example 4

In order to find out the best way of cell disruption, which was carried out before the amount of virus antigen formed was determined, HSV-infected HL-cells were analyzed with ELISA sandwich technique in analogy with example 1, the measurements being carried out on disrupted cells, which had been scraped off, i.e. cell scrap; mechanically homogenized cell scrap; cell scrap homogenized by addition of 0.5% of Triton® X; and after pipetting; after cell disrupting with distilled water according to example 1, and also after freeze-thawing once in the culture medium, which also causes cell disruption but less effectively.

The results are shown in table 5.

TABLE 5
HSV antigen determination after different treatments of infected cells

| | HSV ELISA, After 24-h culture | | A 405 nm After 48-h culture | |
|---|---|---|---|---|
| | low virus dose | high virus dose | low virus dose | high virus dose |
| Aqua dest., freeze thawing and: Cell scrap | 0.395 | 1.400 | 1.370 | 1.240 |
| Cell scrap and homogenization | 0.315 | 0.988 | 1.070 | 0.675 |
| Cell scrap and homogenization in detergent | 0.610 | 1.360 | 1.860 | 1.470 |
| Pipetting | 0.320 | 1.039 | 1.351 | 0.775 |
| Culture medium, freeze-thawing × 1 and: Cell scrap | 0.220 | 0.946 | 0.975 | 0.790 |
| Cell scrap and homogenization | 0.203 | 0.542 | 0.850 | 0.600 |
| Cell scrap and homogenization in detergent | 0.470 | 1.150 | 1.800 | 1.258 |
| Pipetting | 0.210 | 0.700 | 1.060 | 0.750 |
| Only solvent and cells | 0.060—0.095 | | | |

It is apparent from the values in this table that higher absorbance values are obtained in aq. dest. after cell disruption than with cell disruption in the culture medium. Moreover, it is advantageous to scrape off the cells and to homogenize with detergent.

The sensitivity of a virus to different antiviral agents can be graduated by means of the method of the invention. Possibly created resistant virus mutants can also be discovered. The method of the invention is much quicker and more sensitive than a conventional culture method and is based on the disruption of infected cells and establishing small amounts of virus antigen. The invention has been described especially with reference to the virus of the herpes group but the same technique can in principle be used for most viruses. However, one condition is that virus can replicate. This may be an advantage in many situations of treatment, as virus antigen and virus nucleic acid, respectively, can occur in tissue without virus actively replicating (latent virus, killed mature virus).

The amount of the testing material and the virus concentration decide in each individual case how fast the test can be finished and how many antiviral substances can be judged. If the test material is very scarce one cell passage can increase the amount of virus, but the method will then take a corresponding longer time. The same also applies to the conventional technique.

The cell disruption treatment of the invention means that virus antigen can be proved very early and in a relatively simple way. The results can be ready after 1—4 days. The corresponding determination takes at least 7—14 days depending on virus type with the conventional technique described above. Moreover, one advantage is that the invention provides a screening method for virus sensitivity with a comparison of multiple antiviral substances and cell disrupture in combination with immunological determination of antigen not previously described in literature.

When utilizing the invention for diagnostics and determination of sensitivity of herpes virus a kit consisting of plastic plates for cell culture, plastic balls or, alternatively, discs with suitable concentrations of antiviral agents, reagents for ELISA-determination of the respective virus antigen and reagents for cell treatment can preferably be used.

The present determination of virus sensitivity is especially intended to be used when an antiviral treatment is given. An antiviral preparation can be chosen before treatment, during the treatment the effect can be studied as well as the reason for a non-occurring effect be traced, respectively, and guidance can also be obtained for exchange of preparation. At present there is only a limited selection of clinically useful substances, but it can be expected that their number and efficiency will increase. The development is probably to be compared with resistance determination of bacteria, a technique that has become guiding for the choice of antibiotics. However, sensitivity determination of virus is more complicated as alive cells are needed for replication.

Example 5

The following test was carried out in analogy with the method according to Example 1 using the same cells and reagent, if nothing else is indicated. Sterile plastic plates (Linbro, Flow Labs, Hamden, Conn., USA) with 24 wells and $2—3 \times 10^5$ HL-cells per 2.5-cm$^2$ well were used in the VSA-test. The wells were incubated with 0.1 ml of virus dilutions for 30 min. The cells (double sample) were then incubated with 1 ml medium containing different concentrations of the following antiviral substances indicated in table 6. When sera and immunoglobulins are used the dilutions were incubated with virus inoculum for 1 h at 37°C before addition of the cells.

The cells were disrupted in analogy with Example 1 after 1—3 days and the content of virus antigen was decided by means of the ELISA sandwich technique described in this example. The concentrations of antiviral substances which gave 50% of inhibition (IC$_{50}$) after correction for virus inoculum were calculated. The cells incubated with merely antiviral agents or serum gave absorbance values lower than 0.06 at 405 nm.

The ELISA-sandwich test was carried out on microplates (M29AR, Dynatech, Zug, Switzerland) coated with monkey anti-CMV IgG (prepared by immunization with late CMV-antigens from infected MRC-5-cells (human fibroblasts from Medical Research Council, London)). The total antigen amount from 2—4 wells on the plates used in the VSA-test was added to each of the wells on these plates, after which the latter wells were incubated for 2 h at 37°C or 16 h at 4°C. The plates were washed twice and rabbit-anti-CMV DNA polymerase in 100 µl was added to them, after which they were incubated for 2 h at 37°C.

CMV-antigen was thereafter detected in the same way as in Example 1 by incubation (2 h, 37°C) with the same conjugate (0.1 ml pig anti-rabbit IgG conjugated with alkaline phosphatase); inhibition of the reaction; and measurement of the absorbance at 405 nm (A405).

The results are shown in Table 6.

TABLE 6

A 405 nm

| | Antiviral agent | Serum dilution | | | | |
|---|---|---|---|---|---|---|
| | | $10^{-4}$ | $10^{-3}$ | $10^{-2}$ | $10^{-1}$ | $10^0$ |
| 1) | Commercial gammaglobulin | 1.10 | 0.34 | <0.10 | <0.10 | <0.10 |
| 2) | Monkey anti-CMV-nucleocapside antigen | 0.65 | 0.38 | 0.16 | <0.10 | <0.10 |
| 3) | Human anti-CMV-positive serum | 0.80 | 0.97 | 0.46 | <0.10 | <0.10 |
| 4) | CMV-antibody negative serum | — | 1.05 | 0.90 | 0.75 | 0.85 |
| 5) | Rabbit anti-CMV-DNA-polymerase | — | 1.06 | 1.15 | 1.14 | 1.11 |

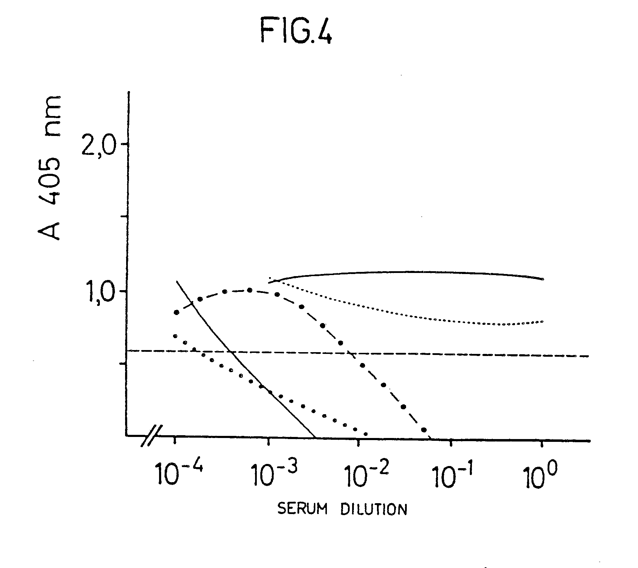

In Fig. 4 the values A 405 nm indicated in table 6 are plotted against the dilution, of which curves shown

"———, the left curve" corresponds to antiviral agent No 1,
".....", corresponds to antiviral agent No 2,
"·-··-·", corresponds to antiviral agent No 3,
"-----", corresponds to antiviral agent No 4 and
"———, the right curve" corresponds to antiviral agent No 5

The level for IC$_{50}$ is designated "----". In fig. 4 computerized 3rd degree curves obtained from Table 6 are illustrated which show that virus antigen production was inhibited by human immunosera and monkey serum but not by rabbit anti-CMV DNA polymerase or human non-immuno serum. In this way the dilutions giving IC$_{50}$ are 1:3125, 1:8925, 1:139, <1:1 and <1:1, resp., for the antiviral agents 1—5.

**Claims**

1. A method of determining the sensitivity of a virus to an anti-viral agent by

a) inoculation of a viral isolate thereof or a material containing such a viable virus mixed with tissue culture medium in batches of cells which permit virus replication;

b) addition of varying concentrations of the antiviral agent to the cells;

c) incubation of the inoculated cells, to which an antiviral agent has been added, and of at least one batch of inoculated cells to which the antiviral agent has not been added, after which the virus is allowed to replicate; and

8

# 0 097 633

d) measurement of the viral replication on the cells, characterized in that, before the measurement d) the cells are disrupted by ultrasonication, hypertonic solutions, mechanical or chemical disintegration and/ or freeze-thawing in the culture medium or after removal of the supernatant followed by addition of distilled water, thereby allowing measurement of the virus antigen content of the cells before or after occurrence of a cytopathic effect in the measurement step d); and in that the measurement d) is carried out by immunological determination of the amount of viral antigen formed through incubation, said determination including a virus antigen-antibody reaction; whereafter

e) the viral sensitivity to the antiviral agent is determined by a comparison of the viral replication obtained by the incubation of the virus cells with addition of the antiviral agent with the viral replication obtained by the incubation of the cells without addition of the antiviral agent.

2. Method according to claim 1, characterized in that the cell disruption is carried out by removing the supernatant and freezing and subsequent thawing of the cells after addition of distilled water.

3. Method according to claim 2, characterized in that the cells are frozen to a temperature of −5°C to −70°C, preferably −20°C, at which temperatures ice crystals form after which the cells are thawed before the immunological determination.

4. Method according to any one of claims 1—3, characterized in that the immunological determination is carried out by enzyme-linked immunosorbent assay (ELISA®), e.g. by inhibition technique but preferably by a sandwich technique.

5. Method according to any one of claims 1—4, characterized in that the antiviral agents are added in a twofold dilution series.

6. Method according to any one of claims 1—5, characterized in that the incubation of cells with virus is interrupted when an optimal virus antigen content is obtained.

7. Method according to any one of claims 1—6, characterized in that, a virus belonging to the herpes virus family is used for inoculation, such as herpes simplex, cytomegalovirus or varicella-zoster virus.

**Patentansprüche**

1. Verfahren zur Bestimmung der Empfindlichkeit eines Virus für ein Antivirales Mittel durch

a) Impfung eines Virusisolats davon oder eines Materials, das ein solches lebendes Virus gemischt mit einem Gewebekulturmedium enhält, in Sätzen von Zellen, worin Viren sich vermehren können;

b) Zusatz von variierenden Konzentrationen vom Antiviralen Mittel zu den Zellen;

c) Bebrütung von den beimpften Zellen, wozu ein Antivirales Mittel zugesetzt wurde, und mindestens von einem Satze der beimpften Zellen, wozu das Antivirales Mittel nicht zugesetzt wurde, wodurch das Virus sich vermehren darf; und

d) Messung der Virusvermehrung in den Zellen, dadurch gekennzeichnet, dass die Zellen vor der Messung d) durch Ultraschallbehandlung, hypertonische Lösungen, mechanische oder chemische Zersetzung und/oder Gefriertauen im Kulturmittel oder nach Entfernung der überstehenden Flüssigkeit mit danach folgendem Zusatz von destilliertem Wasser gesprengt werden, wodurch Messung des Virusantigeninhalts der Zellen vor oder nach dem Auftreten des zytopathischen Effekts in der Messtufe d) erlaubt wird; und dass die Messung d) durch immunologische Béstimmung der durch die Bebrütung gebildeten Virusantigenmenge ausgeführt wird, wobei die Bestimmung eine Virus-Antigen-Antikörper-Reaktion umfasst; wonach

e) die Virusempfindlichkeit für das Antivirales Mittel durch Vergleich der Virusvermehrung bei der Bebrütung der Viruszellen mit Zusatz von dem Antiviralen Mittel mit der Virusvermehrung bei der Bebrütung der Zellen ohne Zusatz vom Antiviralen Mittel bestimmt wird.

2. Verfahren nach Anspruch 1, dadurch gekennzeichnet, dass die Zellsprengung durch Entfernung der überstehenden Flüssingkeit und Gefrieren und folgendes Auftauen der Zellen nach Zusatz von destilliertem Wasser durchgeführt wird.

3. Verfahren nach Anspruch 2, dadurch gekennzeichnet, dass die Zellen bis auf eine Temperatur von −5°C bis −70°C, vorzugsweise −20°C, gefroren werden, bei welchen Temperaturen Eiskristalle gebildet werden, wonach die Zellen vor der immunologischen Bestimmung getaut werden.

4. Verfahren nach einem der Ansprüche 1—3, dadurch gekennzeichnet, dass die immunologische Bestimmung durch enzymgebundene, Immunosorbentanalyse (ELISA®), z.B. Inhibitionstechnik aber vorzugsweise Sanwichtechnik, durchgeführt wird.

5. Verfahren nach einem der Ansprüche 1—4, dadurch gekennzeichnet, dass die Antivirale Mittel in zweifachen Verdünnungsserien zugesetzt werden.

6. Verfahren nach einem der Ansprüche 1—5, dadurch gekennzeichnet, dass die Bebrütung der Zellen mit Virus unterbrochen wird, wenn ein optimaler Virusantigeninhalt erhalten wird.

7. Verfahren nach einem der Ansprüche 1—6, dadurch gekennzeichnet, dass ein Virus, das zur Herpesgruppe gehört, z.B. Herpes Simplex, Zytomegalieviren oder Varizellen-Zosterviren, für die Impfung verwendet wird.

# 0 097 633

**Revendications**

1. Procédé de détermination de la sensibilité d'un virus contre un agent antiviral par:

a) inoculation d'un isolat de ce virus ou d'une substance contenant un tel virus viable, mélangés avec un bouillon de culture de tissus, sur des lots de cellules permettant la réplication virale;

b) addition de l'agent antiviral, en concentrations différentes, aux cellules;

c) incubation des cellules inoculées auxquelles un agent antiviral a été ajouté, ainsi que d'au moins un lot de cellules inoculées auxquelles l'agent antiviral n'a pas été ajouté, après quoi on laisse le virus produire sa réplication; et

d) mesure de la réplication virale sur les cellules; caractérisé en ce que: avant la mesure d), les cellules sont brisées par ultrasons, par des solutions hypertoniques, par désintégration mécanique ou chimique et/ou par gel et dégel, dans le bouillon de culture ou après élimination du liquide surnatant, avec addition suivante d'eau distillée, ce qui permet de mesurer la teneur en antigènes viraux des cellules avant et après l'apparition d'un effet cytopathique à l'étape de mesure d); et en ce que la mesure d) est effectuée par détermination immunologique de la quantité d'antigènes viraux formés par incubation, ladite détermination comprenant une réaction antigène-anticorps viraux; après quoi:

e) la sensibilité virale contre l'agent antiviral est déterminée par comparaison de la réplication virale obtenue par l'incubation des cellules virales avec addition de l'agent antiviral, d'une part, avec celle obtenue par l'incubation des cellules sans addition de l'agent antiviral, d'autre part.

2. Procédé selon la revendication 1, caractérisé en ce que la rupture des cellules est obtenue par élimination du liquide surnatant et par gel et dégel suivant des cellules après addition d'eau distillée.

3. Procédé selon la revendication 2, caractérisé en ce que les cellules sont gelées à une température de −5°C à −70°C, de préférence à −20°C, température à laquelle se forment des cristaux de glace, après quoi les cellules sont dégelées préalablement à la détermination immunologique.

4. Procédé selon une quelconque des revendications 1 à 3, caractérisé en ce que la détermination immunologique est effectuée par un essai immunosorbant à enzymes copulés (ELISA®), par une technique à inhibition par exemple mais de préférence par une technique sandwich.

5. Procédé selon une quelconque des revendications 1 à 4, caractérisé en ce que les agents antiviraux sont ajoutés en une série de dilution double.

6. Procédé selon une quelconque des revendications 1 à 5, caractérisé en ce que l'incubation des cellules à virus est interrompue après obtention d'une teneur optimale en antigènes viraux.

7. Procédé selon une quelconque des revendications 1 à 6, caractérisé en ce qu'un virus appartenant à la famille virale de l'herpès, tel que le virus de l'herpès simplex, le cytomégalovirus ou le virus de la varicelle zostérienne, est utilisé pour l'inoculation.

10

# FIG.1

# FIG.2

# FIG.3

# FIG.4